(19) Europäisches Patentamt
European Patent Office
Office européen des brevets

(11) **EP 3 219 333 A2**

(12) **EUROPEAN PATENT APPLICATION**

(43) Date of publication:
**20.09.2017 Bulletin 2017/38**

(51) Int Cl.:
**A61L 9/04** (2006.01)        **A61Q 13/00** (2006.01)
**C11B 9/00** (2006.01)

(21) Application number: **17157672.1**

(22) Date of filing: **23.02.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **24.02.2016 EP 16305219**

(71) Applicant: **Takasago International Corporation
Tokyo 144-8721 (JP)**

(72) Inventors:
• WARR, Jonathan
  **75017 Paris (FR)**
• PONCELET, Johan
  **75017 Paris (FR)**
• FRASER, Stuart
  **Little Neston, Cheshire CH64 4DH (GB)**

(74) Representative: **Mena, Sandra et al
Cabinet Beau de Loménie
158 rue de l'Université
75340 Paris Cedex 07 (FR)**

(54) **HOUSEHOLD PRODUCT DELIVERING WARMING AND/OR TINGLING SENSATIONS**

(57) The present invention relates to a household product comprising a fragrance composition, said fragrance composition comprising a chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol. In particular, the household product of the invention is an air freshener dispenser device, a floor cleaner, a kitchen or bathroom surface cleaner, a toilet rim block, or a toilet cistern block.

EP 3 219 333 A2

**Description**

Field of the invention

The present invention relates to a household product comprising a fragrance composition, said fragrance composition comprising a chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol, capable of delivering a perceived warming and/or tingling sensation through the air, but without the product *per se* coming into direct contact with the skin or mucous membranes of the subject nor of the product needing to be in close proximity to the nose. In particular, the household product of the invention is an air freshener dispenser device, a floor cleaner, a toilet rim block, or a toilet cistern block. The invention also relates to methods for delivering a perceived warming and/or a perceived tingling sensation through the air.

Background to the invention

Air care products for reducing or masking malodors in the air in homes and enclosed public buildings are commercially available. S. C. Johnson sells products under the GLADE® brand name, Reckitt-Benckiser sells products under the AIR WICK® brand name, and Procter & Gamble sells products under the FEBREZE® and AMBI PUR® brand names. Air freshener compositions have been widely described in the patent literature, for instance in GB 1 315 626; US 6,267,297; US 4,814,212; US 6,036,925; and US 2007/0122373. These products may take several forms as they can be directed devices such as aerosol sprays or trigger sprays which can be applied at the users discretion to specific sites or they can be general air fresheners for a room such as automatic sprays, plug in electrical devices and diffusion devices, such as gels, candles, wax melts or reed diffusers. Another group of vapour-dispensing devices utilizes a carrier material such as paperboard impregnated or coated with a vaporizable composition. Manufacturers are constantly seeking to improve the functionality of these products and add further benefits such as malodour reduction, insect repellency or alternating fragrances to avoid habituation.
Chemaesthesis is defined as the chemical sensibility of the skin and mucous membranes. Chemaesthetic sensations arise when chemical compounds activate receptors associated with other senses that mediate touch and thermal perception. The sensations are usually described as a cooling sensate, a warming sensate, or a tingling sensate.
Chemaesthetic agents may for instance be added to consumer products to enhance the sensory experience. Typical examples of such products are edible and potable compositions, such as:

- beverages, confectionery and chewing gums;
- toiletries such as after-shave lotions, shaving creams, gels and foams, deodorants and antiperspirants, toilet soaps, bath oils and salts, shampoos, hair conditioners, body washes, lipsticks, hair oils, talcum powders, face creams, hand creams, sun-burn lotions, cleansing tissues, dentifrices, and mouthwashes;
- medicinal products and allied compositions including antiseptic ointments, liniments, lotions, decongestants, cough mixtures, throat lozenges, indigestion preparations and oral analgesics.

A wide range of chemaesthetic agents have been developed to deliver sensations appropriate to specific product applications. See for example GB 1315626 and citations thereof which describes a range of low volatility carbon 3 substituted para menthanes as cooling agents, or US 9,890,567 which describes cooling, warming and tingling sensates for their use in food, pharmaceutical, or personal care products.
The known chemaesthetic agents require to be applied directly to the skin or to be used in close proximity to the skin. Many of them have strong odours which may not be entirely pleasant and which may not always blend harmoniously with other fragrance notes. In addition, whilst it is not technically difficult to incorporate chemaesthetic agents into consumer products there are some concerns about possible adverse sensations which might arise. One of the challenges of using chemaesthetic agents in a wider range of consumer products is the varying sensitivity of different parts of the body to a specific concentration of chemaesthetic agent. What is a pleasant warming or tingling sensation at one dosage may become a painful sensation at a higher dosage or if applied to a more sensitive part of the body. For instance, adding a chemaesthetic agent at a dosage that is pleasantly noticeable on a tee-shirt next to the skin of the torso, might become a painful experience if a towel containing the same dosage were used to dry round the eyes. Thus, while there are attempts to add chemaesthetic agents to various consumer products not applied directly to the skin or used in close proximity to the mouth and nose they are generally not formulated to provide a true chemaesthetic effect. Often, the materials are used at a low dosage and merely imply a chemaesthetic effect by association.

Summary of the invention

The present invention provides a household product comprising a fragrance composition based on at least one chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol, all of which are sufficiently volatile so as to evaporate with the fragrance and deliver perceived warming and/or tingling sensation through the air. It has indeed been found that when one of these chemaesthetic agents is formulated into a fragrance composition, a person smelling the composition perceives the air to be warmer and/or perceives a tingling sensation. Household products comprising such a fragrance composition will therefore provide a benefit to consumers. In the context of the present invention, the household product is a non-ingested and a non-topically applied product. Further to avoid being restricted to a narrow range of fragrance notes due to the odour of the chemaesthethic agents themselves, the compounds of the invention are acceptable odour chemaesthetic agents, and are compatible with a wide range of fragrance compositions.

Accordingly, in a first aspect, the invention relates to a household product comprising a fragrance composition, in which said fragrance composition comprises from about 0.01 to less than 10% by weight of at least one chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol, based on the total weight of the fragrance composition.

In another aspect, the invention relates to an air freshener dispenser device, a floor cleaner, a toilet rim block, or a toilet cistern block comprising such a fragrance composition.

The invention also relates to the use of at least one of vanillyl ethyl ether, vanillyl n-propyl ether, vanillyl isopropyl ether, vanillyl butyl ether, or of a household product according to the invention comprising one of these compounds, as an agent for delivering a perceived warming sensation through the air.

The invention also relates to the use of at least one of elemol, elemicin, lime oxide, ocimene quintoxide, 2-isopropenyl-5-methyl-5-vinyltetrahydrofuran, isopulegol, or of a household product according to the invention comprising one of these compounds, as an agent for delivering a perceived tingling sensation through the air.

The invention also relates to a method for delivering a perceived warming sensation through the air by dispersing at least one of vanillyl ethyl ether, vanillyl n-propyl ether, vanillyl isopropyl ether, vanillyl butyl ether, or a household product according to the invention comprising one or more of these compounds, into the air.

Finally, the invention also relates to a method for delivering a perceived tingling sensation through the air by dispersing at least one of elemol, elemicin, lime oxide, ocimene quintoxide, 2-isopropenyl-5-methyl-5-vinyltetrahydrofuran, isopulegol, or a household product according to the invention comprising one or more of these compounds, into the air.

Brief description of the drawings

- **Figure 1** reports the results of the warming perception test of Example 1, and
- **Figure 2** reports the results of the tingling perception test of Example 2.

Detailed description of the invention

According to a first aspect, the invention relates to a household product comprising a fragrance composition, said fragrance composition comprising from about 0.01 to less than 10% by weight, and preferably from about 0.01 to less than 5% by weight, based on the total weight of the fragrance composition, of at least one chemaesthethic agent selected from the group consisting of: vanillyl ethyl ether (CAS 13184-86-6), vanillyl n-propyl ether (CAS 81995-38-2), vanillyl isopropyl ether (CAS 14193-29-4), vanillyl butyl ether (CAS 82654-98-6), elemol (CAS 8024-27-9), elemicin (CAS 487-11-6), lime oxide (CAS 73018-51-6), ocimene quintoxide (citroxide) (CAS 7416-35-5), 2-isopropenyl-5-methyl-5-vinyltetrahydrofuran (CAS 13679-86-2) and isopulegol (CAS 7786-67-6 and CAS 89-79-2).

Elemol (2-(4-ethenyl-4-methyl-3-(prop-1-en-2-yl)cyclohexylpropan-2-ol) exists as several stereochemical isomers depending on whether it is naturally derived or synthetic. In the sense of the invention, the word elemol includes all the stereoisomeric forms of 2-(4-ethenyl-4-methyl-3-(prop-1-en-2-yl)cyclohexylpropan-2-ol. Elemol is found in a variety of plant species such as blood orange, amyris oil, angelica seed oil, baies roses extract, citronella oil, elemi oil, elemi resin, melissa oil, vetiver oil, agarwood, mango leaves extract.

Elemicin is 1,2,3-trimethoxy-5-prop-2-enylbenzene, and is found in a variety of plant extracts such as angelica seed oil, elemi oil, elemi resin, nutmeg absolute, parsley leaf oil, parsley seed oil.

Commercial lime oxide is a mixture of compounds obtained by acidic isomerisation of linalool, which contains ocimene quintoxide (citroxide) (CAS 7416-35-5). 2-isopropenyl-5-methyl-5-vinyltetrahydrofuran (CAS 13679-86-2) can also be obtained by acidic isomerization of linalool. In the sense of the invention, the word lime oxide encompasses all the stereoisomeric forms of ocimene quintoxide and 2-isopropenyl-5-methyl-5-vinyltetrahydrofuran.

The isopulegol of the invention is available either as the racemate (CAS 7786-67-6) or as the (-) isomer (CAS 89-79-2). In one embodiment isopulegol is available as Coolact® P, a product from Takasago. The isopulegol of the invention may have an optical isomer and chemical purity of greater than 90%, preferably greater than 95%, more preferably greater than 97.5%, and even more preferably greater than 99%. Isopulegol purity is determined by gas chromatography using the method described in US 5,773,410 by summing the area percent of impurity peaks and subtracting these from the total measured area which is taken to be 100%.

Some of the chemaesthetic agents of the invention are already known from the prior art to provide warming or tingling effect to the skin by directly stimulating the receptors of the nerve endings of the skin to produce a warming or tingling sensation. For instance, vanillyl butyl ether is known as an active ingredient in products to impart a sharp, tangy bite or a heating/warming sensation (JP 54-67040 and JP 61-9293).

The Inventors have now surprisingly discovered that the chemaesthetic compounds of the invention also produce a perceived warming and/or tingling sensation through the air and can be used in household products containing a fragrance composition. The Inventors have observed that the chemaesthetic compounds of the invention affect the trigeminal nerve - a nerve responsible for sensation in the face, nose and mouth, including pain, temperature and touch - and induce a trigeminal response of the brain. The chemaesthetic agents of the invention thus emerge as being trigeminal-stimulating compounds.

In the sense of the invention, the chemaesthetic agents are agents giving a response when tested against proteins expressed by:

- the TRPV1 gene (Transient Receptor Potential cation channel subfamily V member 1), when the chemaesthetic agent is a warming agent (Turning up the Heat on Pain: TRPV1 Receptors in Pain and Inflammation, Editors: Annika B. Malmberg, Keith R. Bley), and
- the TRPA1 gene (Transient Receptor Potential cation channel subfamily A member 1), when the chemaesthetic agent is a tingling agent (T. Mc Donald et al., Chemesthesis: Chemical Touch in Food and Eating, March 2016, Wiley-Blackwell).

The household product of the invention advantageously comprises a fragrance composition comprising from about 0.01 to about 9 wt%, about 0.01 to about 8 wt%, about 0.01 to about 7 wt%, about 0.01 to about 6 wt%, about 0.01 to about 5 wt%, about 0.01 to about 4 wt%, about 0.01 to about 3 wt%, or about 0.01 to about 2 wt% of at least one chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol, based on the total weight of the fragrance composition. The fragrance composition may comprise from about 0.02 to about 9 wt%, about 0.02 to about 8 wt%, about 0.02 to about 7 wt%, about 0.02 to about 6 wt%, about 0.02 to about 5 wt%, about 0.02 to about 4 wt%, about 0.02 to about 3 wt%, or about 0.02 to about 2 wt% of at least one chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol, based on the total weight of the fragrance composition. The fragrance composition may comprise from about 0.03 to about 9 wt%, about 0.03 to about 8 wt%, about 0.03 to about 7 wt%, about 0.03 to about 6 wt%, about 0.03 to about 5 wt%, about 0.03 to about 4 wt%, about 0.03 to about 3 wt%, or about 0.03 to about 2 wt% of at least one chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol, based on the total weight of the fragrance composition. The fragrance composition may also comprise from about 0.04 to about 9 wt%, about 0.04 to about 8 wt%, about 0.04 to about 7 wt%, about 0.04 to about 6 wt%, about 0.04 to about 5 wt%, about 0.04 to about 4 wt%, about 0.04 to about 3 wt%, or about 0.04 to about 2 wt% of at least one chemaesthethic agent selected from the group consisting of: vanillyl ethyl ether, vanillyl n-propyl ether, vanillyl isopropyl ether, vanillyl butyl ether, elemol, elemicin, lime oxide, ocimene quintoxide, 2-isopropenyl-5-methyl-5-vinyltetrahydrofuran and isopulegol, based on the total weight of the fragrance composition.

According to a first embodiment, the chemaesthetic agent of the invention is a warming agent selected from the group consisting of vanillyl ethyl ether, vanillyl n-propyl ether, vanillyl isopropyl ether and vanillyl butyl ether. Such warming agents are commercially available for example under the names of Hotact® VEE from Takasago for vanillyl ethyl ether, or Hotact® VBE from Takasago for vanillyl buthyl ether.

In this first embodiment, the household product may comprise a fragrance composition which further comprises one or more complementary compound(s) which can be advantageously added to the warming agent, preferably incorporated in a total amount of from 0.5 to 20% by weight, and more preferably from 1 to 10% by weight, based on the total weight of the fragrance composition.

The complementary compound added to the warming agent may advantageously be selected from the group consisting of: 2-ethoxy-4-(hydroxymethyl) phenol (CAS 4912-58-7), vanillin (CAS 121-33-5), ethyl vanillin (CAS 121-32-4), vanillyl isobutyrate (CAS 20665-85-4), veltol (CAS 118-71-8), veltol plus (CAS 4940-11-8), cinnamaldehyde (CAS 104-55-2), vertenex (CAS 32210-23-4), coumarin (CAS 91-64-5), orbitone® (CAS 55464-57-2, CAS 68155-66-8 and CAS 68155-67-9) and bacdanol (Levosandol® from Takasago) (CAS 28219-61-6 and CAS 164203-46-7). According to a preferred embodiment, the warming agent vanillyl ethyl ether, vanillyl n-propyl ether, vanillyl isopropyl ether, or vanillyl butyl ether is combined with 2-ethoxy-4-(hydroxymethyl) phenol.

According to a second embodiment, the chemaesthetic agent of the invention is a tingling agent selected from the group consisting of elemol, elemicin, lime oxide, ocimene quintoxide, 2-isopropenyl-5-methyl-5-vinyltetrahydrofuran and isopulegol, and preferably is a combination of elemol and elemicin. Elemol and elemicin may be added in the fragrance composition as such, or via an essential oil or a natural extract containing them (such as in elemi oil or in elemi resin). Ocimene quintoxide and 2-isopropenyl-5-methyl-5-vinyltetrahydrofuran may also be added in the fragrance composition as such, or via a reaction mixture containing them.

In this second embodiment, the household product may comprise a fragrance composition which further comprises one or more complementary compound(s) which can be advantageously added to the tingling agent, preferably incorporated in a total amount of from 0.1 to 20% by weight, and more preferably from 0.1 to 10% by weight, based on the total weight of the fragrance composition.

The complementary compound added to the tingling agent may advantageously be selected from the group consisting of: citral (CAS 5392-40-5), 1,4-cineole (CAS 470-67-7), gamma-terpinolene (CAS 69073-38-7) and (+)-nootkatone (CAS 4674-50-4).

According to another embodiment, the household product comprises a fragrance composition, which further comprises a perfume ingredient, and preferably at least two, such as at least five, or at least eight distinct perfume ingredients. It can comprise highly complex mixtures of perfume ingredients, chosen to provide any desired odour. The perfume ingredient typically is an olfactively active material. Perfume ingredients typically used in the field of perfumery and suitable for the purposes of the present invention are described more fully in S. Arctander, Perfume Flavors and Chemicals 1969, Vols. I and II, Montclair, N. J. and in The Merck Index, 8th edition, Merck & Co., Inc. Rahway, N. J. The term perfume ingredient encompasses naturally occurring as well as synthetic perfume materials known for use in perfumes, as well as animal oils. A perfume ingredient can also be any natural oil or extract, or chemical compound used in a fragrance composition. Natural oils and extracts are described in The Essential Oils by E. Guenther published in 1949 by Van Nostrand and may include extracts, pressings, collection of exudates, and distillates from any part of suitable plants: roots, rhizomes, bulbs, corms, stem, bark, heartwood, leaves, flowers, seeds and fruit. Examples of such extracts and distillates which include one or more of the complementary tingling materials cited above include citrus fruit oils such as orange, mandarin, grapefruit, lime or lemon oils, tree oils such as pine, or cedarwood, herb oils such as peppermint, thyme, lavender, basil, rosemary, clove or flower extracts such as rose, jasmine muguet, or geranium oil.

It may also be preferred that each perfume ingredient has a molecular weight greater than 100 g/mol, preferably greater than 120 g/mol and lower than 325 g/mol, preferably lower than 300 g/mol. It may further be preferred that each perfume ingredient has a boiling point in the range 80-400°C, such as in the range 100-350°C, when measured at 760 mm Hg.

Advantageously, the perfume ingredient is selected from the following list:

- $C_8$-$C_{18}$ hydrocarbons, preferably delta-3-carene, alpha-pinene, beta-pinene, alpha-terpinene, gamma-terpinene, p-cymene, bisabolene, camphene, caryophyllene, cedrene, farnesene, limonene, longifolene, myrcene, ocimene, valencene, (E,Z)-1,3,5-undecatriene;
- $C_2$-$C_{18}$ aliphatic alcohols, preferably hexanol, octanol, 3-octanol, 2,6-dimethylheptanol, 2-methylheptanol, 2-methyloctanol, (E)-3-hexenol, (E) and (Z)-3-hexenol, 1-octen-3-ol, mixtures of 3,4,5,6,6-pentamethyl-3/4-hepten-2-ol and 3,5,6,6-tetramethyl-4-methyleneheptan-2-ol, (E,Z)-2,6-nonadienol, 3,7-dimethyl-7-methoxyoctan-2-ol, 9-decenol, 10-undecenol, 4-methyl-3-decen-5-ol;
- $C_2$-$C_{18}$ aliphatic aldehydes and their acetals, preferably hexanal, heptanal, octanal, nonanal, decanal, undecanal, dodecanal, tridecanal, 2-methyloctanal, 2-methylnonanal, (E)-2-hexenal, (Z)-4-heptenal, 2,6-dimethyl-5-heptenal, 10-undecenal, (E)-4-decenal, 2-dodecenal, 2,6,10-trimethyl-5,9-undecadienal, heptanal diethyl acetal, 1,1-dimethoxy-2,2,5-trimethyl-4-hexene, citronellyl oxyacetaldehyde;
- $C_3$-$C_{18}$ aliphatic ketones and oximes thereof, preferably 2-heptanone, 2-octanone, 3-octanone, 2-nonanone, 5-methyl-3-heptanone, 5-methyl-3-heptanone oxime, 2,4,4,7-tetramethyl-6-octen-3-one;
- $C_2$-$C_{18}$ aliphatic sulphur-containing compounds, preferably 3-methylthiohexanol, 3-methylthiohexyl acetate, 3-mercaptohexanol, 3-mercaptohexyl acetate, 3-mercaptohexyl butyrate, 3-acetylthiohexyl acetate, 1-menthene-8-thiol;
- $C_2$-$C_{18}$ aliphatic nitrile-containing compounds, preferably 2-nonenenitrile, 2-tridecenenitrile, 2,12-tridecenenenitrile, 3,7-dimethyl-2,6-octadienenitrile, 3,7-dimethyl-6-octenenitrile;
- $C_2$-$C_{18}$ aliphatic carboxylic acids and esters thereof, preferably (E)- and (Z)-3-hexenyl formate, ethyl acetoacetate, isoamyl acetate, hexyl acetate, 3,5,5-trimethylhexyl acetate, 3-methyl-2-butenyl acetate, (E)-2-hexenyl acetate, (E)- and (Z)-3-hexenyl acetate, octyl acetate, 3-octyl acetate, 1-octen-3-yl acetate, ethyl butyrate, butyl butyrate, isoamyl butyrate, hexyl butyrate, (E)- and (Z)-3-hexenyl isobutyrate, hexyl crotonate, ethyl isovalerate, ethyl 2-methylpentanoate, ethyl hexanoate, allyl hexanoate, ethyl heptanoate, allyl heptanoate, ethyl octanoate, ethyl (E,Z)-2,4-decadienoate, methyl 2-octynoate, methyl 2-nonynoate, allyl-2-isoamyloxyacetate, methyl-3,7-dimethyl-2,6-octadienoate;

- $C_4$-$C_{18}$ acyclic terpene alcohols, preferably citronellol, geraniol, nerol, linalool, lavandulol, nerolidol, farnesol, tetrahydrolinalool, tetrahydrogeraniol, 2,6-dimethyl-7-octen-2-ol, 2,6-dimethyloctan-2-ol, 2-methyl-6-methylene-7-octen-2-ol, 2,6-dimethyl-5,7-octadien-2-ol, 2,6-dimethyl-3,5-octadien-2-ol, 3,7-dimethyl-4,6-octadien-3-ol, 3,7-dimethyl-1,5,7-octatrien-3-ol, 2,6-dimethyl-2,5,7-octatrien-1-ol;
- $C_4$-$C_{18}$ acyclic terpene aldehydes and ketones, preferably geranial, neral, citronellal, 7-hydroxy-3,7-dimethyloctanal, 7-methoxy-3,7-dimethyloctanal, 2,6,10-trimethyl-9-undecenal, geranylacetone, and the dimethyl and diethyl acetals of geranial, neral, 7-hydroxy-3,7-dimethyloctanal;
- $C_4$-$C_{18}$ cyclic terpene alcohols, preferably alpha-terpineol, terpineol-4, menthan-8-ol, menthan-1-ol, menthan-7-ol, borneol, isoborneol, linalool oxide, menthol nopol, cedrol, ambrinol, vetiverol, guaiol;
- $C_4$-$C_{18}$ cyclic terpene aldehydes and ketones, preferably fenchone, camphor alpha-ionone, beta-ionone, alpha-n-methylionone, beta-n-methylionone, alpha-isomethylionone, beta-isomethylionone, alpha-irone, alpha-damascone, beta-damascone, beta-damascenone, delta-damascone, gamma-damascone, 1-(2,4,4-trimethyl-2-cyclohexen-1-yl)-2-buten-1-one, 1,3,4,6,7,8a-hexahydro-1,1,5,5-tetramethyl-2H-2,4a-methanonaphthalen-8(5H)-one, menthone nootkatone, dihydronootkatone, alpha-sinensal, beta-sinensal, methyl cedryl ketone;
- $C_4$-$C_{18}$ cyclic alcohols, preferably 4-tert-butylcyclohexanol, 3,3,5-trimethylcyclohexanol, 3-isocamphylcyclohexanol, 2,6,9-trimethyl-Z2,Z5,E9-cyclododecatrien-1-ol, 2-isobutyl-4-methyltetrahydro-2H-pyran-4-ol;
- $C_4$-$C_{18}$ cycloaliphatic alcohols, preferably alpha-3,3-trimethylcyclohexylmethanol, 2-methyl-4-(2,2,3-trimethyl-3-cyclopent-1-yl)butanol, 2-methyl-4-(2,2,3-trimethyl-3-cyclopent-1-yl)-2-buten-1-ol, 2-ethyl-4-(2,2,3-trimethyl-3-cyclopent-1-yl)-2-buten-1-ol, 3-methyl-5-(2,2,3-trimethyl-3-cyclopent-1-yl)-pentan-2-ol, 3-methyl-5-(2,2,3-trimethyl-3-cyclopent-1-yl)-4-penten-2-ol, 3,3-dimethyl-5-(2,2,3-trimethyl-3-cyclopent-1-yl)-4-penten-2-ol, 1-(2,2,6-trimethylcyclohexyl)pentan-3-ol, 1-(2,2,6-trimethylcyclohexyl)hexan-3-ol;
- $C_4$-$C_{18}$ cyclic and cycloaliphatic ethers, preferably cedryl methyl ether, cyclododecyl methyl ether, (ethoxymethoxy)cyclododecane, alpha-cedrene epoxide, 3a,6,6,9a-tetramethyl-dodecahydronaphtho[2,1-b]furan, 3a-ethyl-6,6,9a-trimethyldodecahydro-naphtho[2,1-b]furan, 1,5,9-trimethyl-13-oxabicyclo[10.1.0]trideca-4,8-diene, rose oxide, 2-(2,4-dimethyl-3-cyclohexen-1-yl)-5-methyl-5-(1-methylpropyl)-1,3-dioxane;
- $C_4$-$C_{18}$ cyclic ketones, preferably 4-tert-butylcyclohexanone, 2,2,5-trimethyl-5-pentylcyclopentanone, 2-heptylcyclopentanone, 2-pentylcyclopentanone, 2-hydroxy-3-methyl-2-cyclopenten-1-one, 3-methyl-cis-2-penten-1-yl-2-cyclopenten-1-one, 3-methyl-2-pentyl-2-cyclopenten-1-one, 3-methyl-4-cyclopentadecenone, 3-methyl-5-cyclopentadecenone, 3-methylcyclopentadecanone, 4-(1-ethoxyvinyl)-3,3,5,5-tetramethylcyclohexanone, 4-tert-pentylcyclohexanone, 5-cyclohexadecen-1-one, 6,7-dihydro-1,1,2,3,3-pentamethyl-4(5H)-indanone, 9-cycloheptadecen-1-one, cyclopentadecanone, cyclohexadecanone;
- $C_4$-$C_{18}$ cycloaliphatic aldehydes, preferably 2,4-dimethyl-3-cyclohexenecarbaldehyde, 2-methyl-4-(2,2,6-trimethylcyclohexen-1-yl)-2-butenal, 4-(4-hydroxy-4-methylpentyl)-3-cyclohexenecarbaldehyde, 4-(4-methyl-3-penten-1-yl)-3-cyclohexenecarbaldehyde;
- $C_4$-$C_{18}$ cycloaliphatic ketones, preferably 1-(3,3-dimethylcyclohexyl)-4-penten-1-one, 1-(5,5-dimethyl-1-cyclohexen-1-yl)-4-penten-1-one, 2,3,8,8-tetramethyl-1,2,3,4,5,6,7,8-octahydro-2-naphthalenyl methyl ketone, methyl-2,6,10-trimethyl-2,5,9-cyclododecatrienyl ketone, tert-butyl(2,4-dimethyl-3-cyclohexen-1-yl)ketone;
- esters of cyclic alcohols in $C_4$-$C_{18}$, preferably 2-tert-butylcyclohexyl acetate, 4-tert-butyl-cyclohexyl acetate, 2-tert-pentylcyclohexyl acetate, 4-tert-pentylcyclohexyl acetate, decahydro-2-naphthyl acetate, 3-pentyltetrahydro-2H-pyran-4-yl acetate, decahydro-2,5,5,8a-tetramethyl-2-naphthyl acetate, 4,7-methano-3a,4,5,6,7,7a-hexahydro-5 or 6-indenyl acetate, 4,7-methano-3a,4,5,6,7,7a-hexahydro-5 or 6-indenyl propionate, 4,7-methano-3a,4,5,6,7,7a-hexahydro-5 or 6-indenyl isobutyrate, 4,7-methanooctahydro-5 or 6-indenyl acetate;
- esters of cycloaliphatic carboxylic acids in $C_4$-$C_{18}$, preferably allyl 3-cyclohexylpropionate, allyl cyclohexyloxyacetate, methyl dihydrojasmonate, methyl jasmonate, methyl 2-hexyl-3-oxocyclopentanecarboxylate, ethyl 2-ethyl-6,6-dimethyl-2-cyclohexenecarboxylate, ethyl 2,3,6,6-tetramethyl-2-cyclohexenecarboxylate, ethyl 2-methyl-1,3-dioxolane-2-acetate;
- $C_4$-$C_{18}$ aromatic hydrocarbons, preferably styrene and diphenylmethane;
- $C_4$-$C_{18}$ araliphatic alcohols, preferably benzyl alcohol, 1-phenylethyl alcohol, 2-phenylethyl alcohol, 3-phenylpropanol, 2-phenylpropanol, 2-phenoxyethanol, 2,2-dimethyl-3-phenylpropanol, 2,2-dimethyl-3-(3-methylphenyl)propanol, 1,1-dimethyl-2-phenylethyl alcohol, 1,1-dimethyl-3-phenylpropanol, 1-ethyl-1-methyl-3-phenylpropanol, 2-methyl-5-phenylpentanol, 3-methyl-5-phenylpentanol, 3-phenyl-2-propen-1-ol, 4-methoxybenzyl alcohol, 1-(4-isopropylphenyl)ethanol;

- esters of araliphatic alcohols in C$_4$-C$_{18}$ and aliphatic carboxylic acids in C$_4$-C$_{18}$, preferably benzyl acetate, benzyl propionate, benzyl isobutyrate, benzyl isovalerate, 2-phenylethyl acetate, 2-phenylethyl propionate, 2-phenylethyl isobutyrate, 2-phenylethyl isovalerate, 1-phenylethyl acetate, alpha-trichloromethylbenzyl acetate, alpha,alpha-dimethylphenylethyl acetate, alpha,alpha-dimethylphenylethyl butyrate, cinnamyl acetate, 2-phenoxyethyl isobutyrate, 4-methoxybenzyl acetate;
- C$_2$-C$_{18}$ araliphatic ethers, preferably 2-phenylethyl methyl ether, 2-phenylethyl isoamyl ether, 2-phenylethyl 1-ethoxyethyl ether, phenylacetaldehyde dimethyl acetal, phenylacetaldehyde diethyl acetal, hydratropaldehyde dimethyl acetal, phenylacetaldehyde glycerol acetal, 2,4,6-trimethyl-4-phenyl-1,3-dioxane, 4,4a,5,9b-tetrahydroindeno[1,2-d]-m-dioxin, 4,4a,5,9b-tetrahydro-2,4-dimethylindeno[1,2-d]-m-dioxin;
- C$_4$-C$_{18}$ aromatic and araliphatic aldehydes, preferably benzaldehyde, phenylacetaldehyde, 3-phenylpropanal, hydratropaldehyde, 4-methylbenzaldehyde, 4-methylphenylacetaldehyde, 3-(4-ethylphenyl)-2,2-dimethylpropanal, 2-methyl-3-(4-isopropylphenyl)propanal, 2-methyl-3-(4-tert-butylphenyl)propanal, 3-(4-tert-butylphenyl)propanal, cinnamaldehyde, alpha-butylcinnamaldehyde, alpha-amylcinnamaldehyde, alpha-hexylcinnamaldehyde, 3-methyl-5-phenylpentanal, 4-methoxybenzaldehyde, 4-hydroxy-3-methoxybenzaldehyde, 4-hydroxy-3-ethoxybenzaldehyde, 3,4-methylenedioxybenzaldehyde, 3,4-dimethoxybenzaldehyde, 2-methyl-3-(4-methoxyphenyl)propanal, 2-methyl-3-(4-methylenedioxyphenyl)propanal;
- C$_4$-C$_{18}$ aromatic and araliphatic ketones, preferably acetophenone, 4-methylacetophenone, 4-methoxyacetophenone, 4-tert-butyl-2,6-dimethylacetophenone, 4-phenyl-2-butanone, 4-(4-hydroxyphenyl)-2-butanone, 1-(2-naphthalenyl)ethanone, benzophenone, 1,1,2,3,3,6-hexamethyl-5-indanyl methyl ketone, 6-tert-butyl-1,1-dimethyl-4-indanyl methyl ketone, 1-[2,3-dihydro-1,1,2,6-tetramethyl-3-(1-methylethyl)-1H-5-indenyl]ethanone, 5',6',7',8'-tetrahydro-3',5',5',6',8',8'-hexamethyl-2-acetonaphthone;
- C$_4$-C$_{18}$ aromatic and araliphatic carboxylic acids and esters thereof, preferably phenylacetic acid, methyl benzoate, ethyl benzoate, hexyl benzoate, benzyl benzoate, methyl phenylacetate, ethyl phenylacetate, geranyl phenylacetate, phenylethyl phenylacetate, methyl cinnamate, ethyl cinnamate, benzyl cinnamate, phenylethyl cinnamate, cinnamyl cinnamate, allyl phenoxyacetate, methyl salicylate, isoamyl salicylate, hexyl salicylate, cyclohexyl salicylate, cis-3-hexenyl salicylate, benzyl salicylate, phenylethyl salicylate, methyl 2,4-dihydroxy-3,6-dimethylbenzoate, ethyl 3-phenylglycidate, ethyl 3-methyl-3-phenylglycidate;
- nitrogen-containing aromatic compounds in C$_4$-C$_{18}$, preferably 2,4,6-trinitro-1,3-dimethyl-5-tert-butylbenzene, 3,5-dinitro-2,6-dimethyl-4-tert-butylacetophenone, cinnamonitrile, 5-phenyl-3-methyl-2-pentenenitrile, 5-phenyl-3-methylpentanenitrile, methyl anthranilate, methyl N-methylanthranilate, Schiff bases of methyl anthranilate with 7-hydroxy-3,7-dimethyloctanal, 2-methyl-3-(4-tert-butylphenyl)propanal, 2,4-dimethyl-3-cyclohexene-carbaldehyde, 6-isopropylquinoline, 6-isobutylquinoline, 6-sec-butylquinoline, indole, skatole, 2-methoxy-3-isopropylpyrazine, 2-isobutyl-3-methoxypyrazine;
- phenols, phenyl ethers and phenyl esters, preferably estragole, anethole, eugenol, eugenyl methyl ether, isoeugenol, isoeugenyl methyl ether, thymol, carvacrol, diphenyl ether, beta-naphthyl methyl ether, beta-naphthyl ethyl ether, beta-naphthyl isobutyl ether, 1,4-dimethoxybenzene, eugenyl acetate, 2-methoxy-4-methylphenol, 2-ethoxy-5-(1-propenyl)phenol, p-cresyl phenylacetate;
- heterocyclic compounds in C$_4$-C$_{12}$, preferably 2,5-dimethyl-4-hydroxy-2H-furan-3-one, 2-ethyl-4-hydroxy-5-methyl-2H-furan-3-one, 3-hydroxy-2-methyl-4H-pyran-4-one, 2-ethyl-3-hydroxy-4H-pyran-4-one;
- lactones in C$_4$-C$_{18}$, preferably 1,4-octanolide, 3-methyl-1,4-octanolide, 1,4-nonanolide, 1,4-decanolide, 8-decen-1,4-olide, 1,4-undecanolide, 1,4-dodecanolide, 1,5-decanolide, 1,5-dodecanolide, 1,15-pentadecanolide, cis and trans-11-pentadecen-1,15-olide, cis- and trans-12-pentadecen-1,15-olide, 1,16-hexadecanolide, 9-hexadecen-1,16-olide, 10-oxa-1,16-hexadecanolide, 11-oxa-1,16-hexadecanolide, 12-oxa-1,16-hexadecanolide, ethylene 1,12-dodecanedioate, ethylene 1,13-tridecanedioate, coumarin, 2,3-dihydrocoumarin, octahydrocoumarin.

The perfume ingredient present in the fragrance composition of the household product preferably does not contain ionizing functional groups, such as sulfonates, sulphates, phosphates or quaternary ammonium ions.

The fragrance compositions of the invention may include one or more support materials, such as solvents or UV stabilizers. The amount of solvent(s) may represent up to 60% by weight, such as up to 50% by weight, such as up to 40% by weight, of the total weight of the household product. Examples of suitable solvents include hydrocarbons such as those sold under the trade name Isopar®, ethers such as those sold under the trade name Dowanol®, benzyl benzoate, isopropyl myristate, dialkyl adipates, dialkyl succinates, dialkyl glutarates (such as the dimethyl esters sold under the trade name Flexisolv®), citrate esters (such as triethyl citrate and acetyl tributyl citrate), soybean methyl ester such as ME-S1885 (sold by Peter Cremer NA), diethyl phthalate, diethylene glycol monoethyl ether, 3-methoxy-3-methyl-1-butanol, dipropylene glycol and isopropylidene glycerol sold under the trade name Augeo® Clean Multi. Examples of UV stabilisers include butyl methoxy dibenzoyl methane sold as Parsol® 1789, bis-ethylhexyloxyphenolmethoxyphenyl triazine, those sold under the trade name Uvinol® such as Uvinol D50 (bis(2,4 dihydroxyphenyl)methanone), Uvinol MC80 (octyl methoxycinnamate) and Uvinol M40 (benzophenone-3).

In one embodiment, the household product of the invention is an air freshener dispenser device.

The air freshener dispenser device can be:

- a passive evaporative device selected from sprayers (such as automatic sprays, hand operated pressurised aerosol sprays or pump action sprays, as illustrated in US 2007/0122373), diffusers, gels, candles, waxes, wax melts, pot-pourri, impregnated papers and laminated cardboard, or
- an electrically powered air freshener dispenser device, preferably comprising a heating element, said electrically powered air freshener dispenser device being preferably a piezo-electric device or a plug, as described in US 6,123,935.

According to another aspect, the household product of the invention is a kitchen or bathroom surface cleaner or a floor cleaner. Floor cleaners, also known as general purpose cleaner, also include carpet cleaner. They may be in several forms: isotropic liquids, thickened liquids with or without abrasive, pastes, gels, foams or sprays. They can be used directly from the bottle or after dilution in water. Various delivery methods have been devised for the convenience of the users, some are sprayed onto surfaces from trigger spray bottles, or alternatively they can be poured directly onto surfaces. They may contain additional ingredients such as acids for limescale removal, biocides for hygiene, or bleaching agents. A standard floor cleaner composition is given in Table 1 below, which summarises the main ingredients and their quantities (taken from Surfactant Science Series Vol. 67 Liquid Detergents chapter on Speciality Liquid Household Surface Cleaners p. 479, Table 4).

Table 1: Standard floor cleaner composition

| Categories of ingredients | Example of ingredients | Amount (% by weight) |
|---|---|---|
| Anionic surfactant | Alkylbenzene sulfonate as supplied by Shell as Dobs 055, alkane sulphonate as Hostaspur SAS60 | 0-35 |
| Nonionic surfactant | Ethoxylated alcohol as Neodol 9-11 6EO, mixed ethoxy/propoxy alcohol as the Pluronic® series from BASF, amine oxide, alkanolamides and betaines | 1-35 |
| Hydrotropes | Sodium cumene sulphonate, xylene sulphonate | 0-10 |
| Builder/sequestrant | Citrates, EDTA salts, phosphonate salts, lactic acid and polyacrylates | 0-10 |
| Solvent | Lower alcohols, glycol ethers, benzyl alcohol, hydrocarbons such as limonene | 0.5-50 |
| Disinfectant | Hypochlorite bleach, pine oil, alcohols in $C_1$-$C_6$, quaternary ammonium salts | 0-15 |
| Perfume, colour, thickening polymer, sequestrant, preservatives | - | 0.1-3 |
| Water | - | up to 100 |

According to another aspect, the household product of the invention is a solid or liquid toilet rim block.

The solid toilet rim blocks are intended to be located under the rim of a lavatory bowl or urinal such that, during a flushing cycle, water from the cistern flows over the block thereby dissolving a portion of the toilet rim block. The invention also relates to cageless rim blocks which adhere directly to the surface of the lavatory pan, and to solid toilet cistern blocks which are placed in the cistern and dissolved slowly in the water contained therein. It will be appreciated that the solubility characteristics of these two products are quite different, since one is constantly under water while the other has intermittent short term contact with water. However, they both contain a surfactant, fillers, and a fragrance composition as defined according to the invention, and optionally bleaching agents, germicides and anti-limescale agents. Typical formulations are described in EP 0 462 643, GB 2 178 442, US 8,658,588 and US 4,874,536, which are incorporated herein by reference.

The liquid toilet rim blocks are devices that dispense liquid compositions directly into a lavatory bowl from under the rim of said bowl. Such liquid toilet rim blocks are usually attached by various means, such as hooks and the like, to the rim of the lavatory bowl. Every time a toilet equipped with a liquid toilet rim block is flushed, an amount of composition is dispensed into the lavatory bowl. Examples of liquid toilet rim blocks are given in WO 02/40792, EP 0 775 741 and WO 01/94520, which are incorporated herein by reference.

According to a second aspect, the invention relates to the use of vanillyl ethyl ether, vanillyl n-propyl ether, vanillyl isopropyl ether, vanillyl butyl ether, or of a household product as defined according to the invention comprising one of these warming agents, as an agent for delivering a perceived warming sensation through the air.

According to a third aspect, the invention relates to the use of elemol, elemicin, lime oxide, ocimene quintoxide, 2-isopropenyl-5-methyl-5-vinyltetrahydrofuran, isopulegol, or of a household product as defined according to the invention comprising one of these tingling agents, as an agent for delivering a perceived tingling sensation through the air.

According to a fourth aspect, the invention provides a method for delivering a perceived warming and tingling sensation through the air which comprises dispersing:

- at least one warming agent selected from the group consisting of: vanillyl ethyl ether (CAS 13184-86-6), vanillyl n-propyl ether (CAS 81995-38-2), vanillyl isopropyl ether (CAS 14193-29-4), vanillyl butyl ether (CAS 82654-98-6), simultaneously with
- at least one tingling agent selected from the group consisting of: elemol (CAS 8024-27-9), elemicin (CAS 487-11-6), lime oxide (CAS 73018-51-6), ocimene quintoxide (CAS 7416-35-5), 2-isopropenyl-5-methyl-5-vinyltetrahydrofuran (CAS 13679-86-2), isopulegol (CAS 7786-67-6 and CAS 89-79-2),

or a household product as defined according to the invention comprising both a warming and a tingling agents, into the air.

According to a fifth aspect, the invention provides a method of delivering a perceived warming sensation through the air which comprises dispersing vanillyl ethyl ether, vanillyl n-propyl ether, vanillyl isopropyl ether, vanillyl butyl ether, or a household product as defined according to the invention comprising at least one of these warming agents, into the air.

Finally, the invention provides a method of delivering a perceived tingling sensation through the air which comprises dispersing elemol, elemicin, lime oxide, ocimene quintoxide, 2-isopropenyl-5-methyl-5-vinyltetrahydrofuran, isopulegol, or a household product as defined according to the invention comprising at least one of these tingling agents, into the air.

The household product can be dispersed by any of the sprayers, articles and devices described herein, or by any other suitable device, or in any other suitable manner. The household product can be dispersed in the form of spray droplets.

In addition to the above provisions, the invention also comprises other provisions which will emerge from the remainder of the description which follows.

**Examples:**

Example 1: Perception test for warming agents

A panel of 20 subjects was selected to take part in a comparative test between capsaicin (reference) (Sigma-Aldrich), vanillyl butyl ether (Hotact® VBE) and vanillyl ethyl ether (Hotact® VEE). The reference capsaicin is an active component of chili peppers known for its warming effect on the skin/the tongue (S. Kosuge et al., J. Agric. Chem. Soc., 1962, 36, p.51).

The samples were prepared by dilution of the pure raw materials at 1% in Triethyl Citrate (TEC).

In a first part of the evaluation, the subjects of the panel smell the samples one by one and rate them on the following scale:

- warming perception in the nose: 0 (not at all) to 100 (very strong sensation).

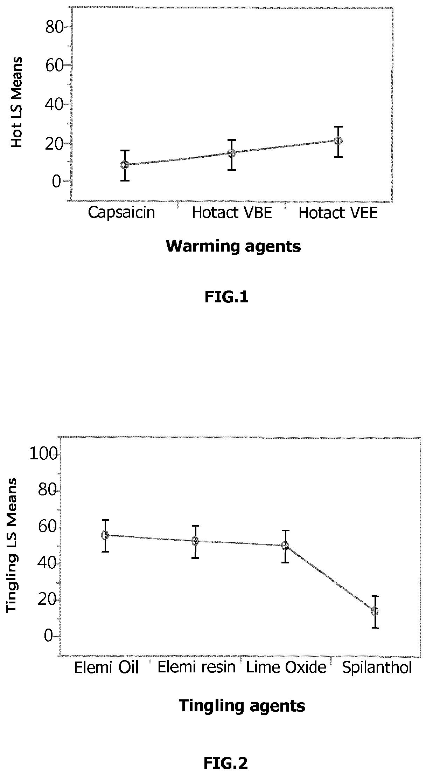

The results of the warming perception test are shown in Figure 1.

The ANOVA test and Student's t-test both show that the subjects of the panel feel an improved warming perception for vanillyl ethyl ether (Hotact® VEE) and vanillyl butyl ether (Hotact® VBE), compared to the reference capsaicin. These results are statistically significant (ANOVA: F ratio = 15.12; $p < 0.001$; t-test: alpha = 0.05).

In a second part of the evaluation, the subjects of the panel rank the chemaesthetic agents from the less warming agent (rank 1) to the more warming agents (rank 4). The mean values obtain for the ranking where as follows:

- Capsaicin: Mean rank = 1.650,
- Hotact® VEE: Mean rank = 2.450, and
- Hotact® VBE: Mean rank = 2.925.

The Friedman test shows that the materials are ranked differently ($p < 0.01$).

A Nemenyi multiple comparison test indicates that vanillyl ethyl ether (Hotact® VEE) and vanillyl butyl ether (Hotact® VBE) are significantly more warming than the reference capsaicin.

Example 2: Perception test for tingling agents

A panel of 20 subjects was selected to take part in a comparative test between elemi oil, elemi resin, lime oxide and spilanthol (reference) (Takasago). The reference spilanthol is contained in jambu and known for its tingling effect on the skin/the tongue (US 9,890,567; J. Paulraj et al., Advances in Pharmacological Sciences, Vol. 2013, Article ID 510298, 22 pages).

The samples were prepared as solutions of the pure raw materials at 1% in Triethyl Citrate (TEC), and portions decanted into small jars for testing.

In a first part of the evaluation, the subjects of the panel smell the samples one by one and rate them on the following scale:

- tingling perception in the nose: 0 (not at all) to 100 (very strong sensation).

The results of the tingling perception test are shown in Figure 2.

The ANOVA test and Student's t-test both show that the subjects of the panel feel an improved tingling perception for elemi oil, elemi resin and lime oxide, compared to the reference spilanthol. These results are statistically significant (ANOVA: F ratio = 19.29; p < 0.001; t-test: alpha = 0.05). The Student's t-test shows that spilanthol is clearly the less tingling agent, with little effect at all, whereas the three tingling agents of the invention are very tingling.

In a second part of the evaluation, the subjects of the panel rank the chemaesthetic agents from the less tingling agent (rank 1) to the more tingling agents (rank 4). The mean values obtain for the ranking where as follows:

- Spilanthol: Mean rank = 1.150,
- Elemi resin: Mean rank = 2.250,
- Elemi oil: Mean rank = 3.250, and
- Lime oxide: Mean rank = 3.350.

The Friedman test shows that the materials are ranked differently (p < 0.001).

A Nemenyi multiple comparison test indicates that elemi resin, elemi oil and lime oxide are perceived as significantly more tingling than the reference spilanthol.

Example 3: Comparison of tingling effect between isopulegol and menthoxypropanediol

A panel of 80 subjects was selected to take part in a comparative test between isopulegol (available as Coolact® P) and menthoxypropanediol (CAS 87061-04-9, available as Coolact® 10).

The samples of isopulegol and menthoxypropanediol were prepared by dilution to 3% in Triethyl Citrate (TEC).

The subjects had to score whether they perceived a tingling sensation in the nose, on a scale of 0 (no sensation) to +100 (extremely tingling).

The ANOVA test and Student's t-test both showed that the subjects of the panel felt an improved tingling perception for isopulegol (available as Coolact® P) compared to menthoxypropanediol (Coolact® 10). These results are statistically significant (ANOVA: F ratio = 44.80; p < 0.05; t-test: alpha = 0.05).

Surprisingly, the results show that Coolact® 10 (known as a cooling agent on the skin) leads to a lower tingling sensation compared to isopulegol (Coolact® P).

Example 4: Tingling perception test in a fragrance

A panel of 20 subjects was selected to take part in a comparative test between a fragrance F2 and another fragrance F1, enriched with elemi oil, lime oxide or isopulegol. The solutions were prepared as 3% solutions in Triethyl Citrate (TEC).

The subjects separately had to sniff the samples one after another when presented in a random order and score on a scale of 0 (no sensation at all) to 100 (very strong sensation), how intense a tingling sensation was perceived.

Fragrance formula F1

| CAS number | Ingredients | wt% |
|---|---|---|
| 25265-71-8 | Dipropylene glycol | 37.6 |

(continued)

| CAS number | Ingredients | wt% |
|---|---|---|
| 2705-87-5 | Allyl cyclohexyl propionate | 0.81 |
| 28940-11-6 | Calone 1951 | 0.05 |
| 8008-56-8 | Lemon oils natural | 15.35 |
| 8008-57-9 | Orange oil Brazilian | 10.87 |
| 78-70-6 | Linalool | 6.79 |
| 88-41-5 | Verdox | 5.43 |
| 106-22-9 | Citronellol 950 | 4.07 |
| 115-95-7 | Linalyl acetate | 8.15 |
| 140-11-4 | Benzyl acetate | 2.72 |
| 142-92-7 | Hexyl acetate | 2.04 |
| 93-92-5 | Styrallyl acetate | 2.04 |
| 98-55-5 | Terpineol 900 alpha | 1.36 |
| 18479-58-8 | Dihydromyrcenol | 1.36 |
| 63500-71-0 | Florol | 1.36 |
|  |  |  |
|  | Total: | 100.00 |

Fragrance formula F2:

| CAS number | Ingredients | wt% |
|---|---|---|
| 6790-58-5 | Ambroxan | 0.30 |
| 123-11-5 | Anisic aldehyde | 1.78 |
| 100-79-8 | 2,2-Dimethyl-4-hydroxymethyl-1,3-dioxolane | 56.80 |
| 140-11-4 | Benzyl acetate | 3.56 |
| 100-52-7 | Benzaldehyde | 1.78 |
| 91-64-5 | Coumarin | 1.78 |
| 2305-05-7 | Decalactone, gamma | 3.56 |
| 151-05-3 | Dimethyl benzyl carbonyl acetate | 2.37 |
| 25265-71-8 | Dipropylene glycol | 10.00 |
| 63500-71-0 | Florol 966458 | 2.13 |
| 24851-98-7 | Hedione | 1.48 |
| 78-70-6 | Linalool syn | 7.11 |
| 39255-32-8 | Manzanate | 1.19 |
| 27939-60-2 | Triplal | 0.47 |
| 104-67-6 | Undecalactone, gamma (ALD C-14) | 4.27 |
| 4940-11-8 | Veltol plus | 1.42 |
|  |  |  |
|  | Total: | 100.00 |

Fragrance formula F1-A + elemi oil

| CAS number | Ingredients | wt% |
|---|---|---|
| 25265-71-8 | Dipropylene glycol | 36.8 |
| 2705-87-5 | Allyl cyclohexyl propionate | 0.81 |
| 28940-11-6 | Calone 1951 | 0.05 |
| 8008-56-8 | Lemon oils natural | 15.35 |
| 8008-57-9 | Orange oil Brazilian | 10.87 |
| 78-70-6 | Linalool | 6.79 |
| 88-41-5 | Verdox | 5.43 |
| 106-22-9 | Citronellol 950 | 4.07 |
| 115-95-7 | Linalyl acetate | 8.15 |
| 140-11-4 | Benzyl acetate | 2.72 |
| 142-92-7 | Hexyl acetate | 2.04 |
| 93-92-5 | Styrallyl acetate | 2.04 |
| 98-55-5 | Terpineol 900 alpha | 1.36 |
| 18479-58-8 | Dihydromyrcenol | 1.36 |
| 63500-71-0 | Florol | 1.36 |
| 8023-89-0 | Elemi oil | 0.8 |
| | | |
| | Total: | 100.00 |

Fragrance formula F1-B + lime oxide

| CAS number | Ingredients | wt% |
|---|---|---|
| 25265-71-8 | Dipropylene glycol | 37.30 |
| 2705-87-5 | Allyl cyclohexyl propionate | 0.81 |
| 28940-11-6 | Calone 1951 | 0.05 |
| 8008-56-8 | Lemon oils natural | 15.35 |
| 8008-57-9 | Orange oil Brazilian | 10.87 |
| 78-70-6 | Linalool | 6.79 |
| 88-41-5 | Verdox | 5.43 |
| 106-22-9 | Citronellol 950 | 4.07 |
| 115-95-7 | Linalyl acetate | 8.15 |
| 140-11-4 | Benzyl acetate | 2.72 |
| 142-92-7 | Hexyl acetate | 2.04 |
| 93-92-5 | Styrallyl acetate | 2.04 |
| 98-55-5 | Terpineol 900 alpha | 1.36 |
| 18479-58-8 | Dihydromyrcenol | 1.36 |
| 63500-71-0 | Florol | 1.36 |
| 73018-51-6 | Lime oxide | 0.30 |

(continued)

| CAS number | Ingredients | wt% |
|---|---|---|
|  |  |  |
|  | Total: | 100.00 |

Fragrance formula F1-C + isopulegol

| CAS number | Ingredients | wt% |
|---|---|---|
| 25265-71-8 | Dipropylene glycol | 36.6 |
| 2705-87-5 | Allyl cyclohexyl propionate | 0.81 |
| 28940-11-6 | Calone 1951 | 0.05 |
| 8008-56-8 | Lemon oils | 15.35 |
| 8008-57-9 | Orange oil Brazilian | 10.87 |
| 78-70-6 | Linalool | 6.79 |
| 88-41-5 | Verdox | 5.43 |
| 106-22-9 | Citronellol 950 | 4.07 |
| 115-95-7 | Linalyl acetate | 8.15 |
| 140-11-4 | Benzyl acetate | 2.72 |
| 142-92-7 | Hexyl acetate | 2.04 |
| 93-92-5 | Styrallyl acetate | 2.04 |
| 98-55-5 | Terpineol 900 alpha | 1.36 |
| 18479-58-8 | Dihydromyrcenol | 1.36 |
| 63500-71-0 | Florol | 1.36 |
| 7786-67-6 | Isopulegol | 1.00 |
|  |  |  |
|  | Total: | 100.00 |

The mean panel scores were as follow:

| Sample | Tingling perception score |
|---|---|
| Fragrance 2 (F2) | 19.40 |
| F1-A + elemi oil | 45.25 |
| F1-B + lime oxide | 46.40 |
| F1-C + isopulegol | 44.05 |

An ANOVA analysis performed on this data with fragrance and panellist as factor and the warming score as a variable showed a significant result (ANOVA: F ratio = 9.12; $p < 0.01$). The multiple comparison test Student's T-test showed that both mixture with F2 were perceived warmer than F1 (t-test: alpha = 0.05).

Example 5: Warming perception test in a fragrance

A panel of 20 subjects was selected to take part in a comparative test between a fragrance F1 as defined in Example 4 and another fragrance F2, enriched with vanillyl ethyl ether (Hotact[®] VEE) or vanillyl butyl ether (Hotact[®] VBE) . The solution where prepared at 3% in TEC.

The subject had to sniff the samples one after another when presented in a random order and score on a scale of 0 (no sensation at all) to 100 (very strong sensation), if the sample where warming or not.

Fragrance formula F2-A + vanillyl ethyl ether

| CAS number | Ingredients | wt% |
|---|---|---|
| 6790-58-5 | Ambroxan | 0.30 |
| 123-11-5 | Anisic aldehyde | 1.78 |
| 100-79-8 | 2,2-Dimethyl-4-hydroxymethyl-1,3-dioxolane | 56.20 |
| 140-11-4 | Benzyl acetate | 3.56 |
| 100-52-7 | Benzaldehyde | 1.78 |
| 91-64-5 | Coumarin | 1.78 |
| 2305-05-7 | Decalactone, gamma | 3.56 |
| 151-05-3 | Dimethyl benzyl carbonyl acetate | 2.37 |
| 25265-71-8 | Dipropylene glycol | 10.00 |
| 63500-71-0 | Florol 966458 | 2.13 |
| 24851-98-7 | Hedione | 1.48 |
| 13184-86-6 | Vanillyl ethyl ether | 0.60 |
| 78-70-6 | Linalool syn | 7.11 |
| 39255-32-8 | Manzanate | 1.19 |
| 27939-60-2 | Triplal | 0.47 |
| 104-67-6 | Undecalactone, gamma (ALD C-14) | 4.27 |
| 4940-11-8 | Veltol plus | 1.42 |
|  |  |  |
|  | Total: | 100.00 |

Fragrance formula F2-B + vanillyl butyl ether

| CAS number | Ingredients | wt% |
|---|---|---|
| 6790-58-5 | Ambroxan | 0.30 |
| 123-11-5 | Anisic aldehyde | 1.78 |
| 100-79-8 | 2,2-Dimethyl-4-hydroxymethyl-1,3-dioxolane | 56.50 |
| 140-11-4 | Benzyl acetate | 3.56 |
| 100-52-7 | Benzaldehyde | 1.78 |
| 91-64-5 | Coumarin | 1.78 |
| 2305-05-7 | Decalactone, gamma | 3.56 |
| 151-05-3 | Dimethyl benzyl carbonyl acetate | 2.37 |
| 25265-71-8 | Dipropylene glycol | 10.00 |
| 63500-71-0 | Florol 966458 | 2.13 |
| 24851-98-7 | Hedione | 1.48 |
| 82654-98-6 | Vanillyl buty ether | 0.30 |
| 78-70-6 | Linalool syn | 7.11 |
| 39255-32-8 | Manzanate | 1.19 |

(continued)

| CAS number | Ingredients | wt% |
|---|---|---|
| 27939-60-2 | Triplal | 0.47 |
| 104-67-6 | Undecalactone, gamma (ALD C-14) | 4.27 |
| 4940-11-8 | Veltol plus | 1.42 |
| | | |
| | Total: | 100.00 |

The mean of the scoring were as follow:

| Sample | Tingling perception score |
|---|---|
| Fragrance 1 (F1) | 10.7 |
| F2-A + vanillyl butyl ether | 20.60 |
| F2-B + vanillyl ethyl ether | 24.90 |

An ANOVA analysis performed on this data with fragrance and panellist as factor and the warming score as a variable showed a significant result (ANOVA: F ratio = 4.70; $p < 0.05$). The multiple comparison test Student's t-test showed that both mixtures with F2 were perceived warmer than F1 (t-test: alpha = 0.05).

Example 6: Tests for TRPA1 and TRPV1 responses

The following are the test protocols used in determining whether fragrance ingredients show a response on the TRPA1 and TRPV1 receptors which are associated with tingling and warming sensations respectively. In all cases the reaction is measured by fluorescence imaging using high throughput screening equipment.

*Method for Screening on Receptors TRPA1:*

HEK-293 (Human Embryonic Kidney) cells stably transfected with human TRPA1 were grown in a culture medium of EMEM (medium Essential Medium Eagle with Earl's salts balanced salt solution (Lonza catalogue No BE12-125F), 5 mL of 200 mM Ultraglutamine 1 (Lonza Catalogue No BE17-605E/U1), 5 mL of 100X Penicillin/Streptomycin (Lonza Catalogue No DE17-602E) 50 mL of Fetal bovine serum (Euroclone Cat No ECS0180L) 2 mL of 100 mg/mL Genticin sulphate (G418 sulphate InvivoGen CatAnt-gn-5).

10,000 cells/well were seeded in a 384 MTP in complete medium. Then, after 24 hours, the culture medium was manually removed. The cells were loaded with 20 $\mu$L/well of 0.5x Fluo-8 NW dye in standard Tyrode's buffer (Screen Quest™ Fluo-8 No wash Calcium assay kit supplied by AAT Bioquest catalogue No 26316). The plate was incubated for 1 hour at room temperature in the dark. 10 $\mu$L/well of test chemaesthetic agents were injected and controlled with the FLIPR$^{TETRA}$ (Molecular Devices). The emitted fluorescence signal at 520 nm was recorded for 3 minutes. The compound multiwall plate contained the samples at 8 different doses in quadruplicate data points. Dose response of the samples were performed starting from 100 mM concentration in 100% DMSO with a serial dilution of 1:2 and then diluted in assay buffer at the final concentration of 100 $\mu$M, 50 $\mu$M, 25 $\mu$M, 12.5 $\mu$M, 6 $\mu$M, 3 $\mu$M, 1.5 $\mu$M, and 0.7 $\mu$M.

In the compound plate were also present a dose response of the reference agonist at 8 concentrations and in duplicate. Spilanthol was the reference agonist for TRPA1 in this study. These reference controls dosed as 16 wells (duplicates for each concentration) of reference agonist at $EC_{100}$ corresponded to the MAX signal response (100% activity). There were also 16 wells of blanks, *i.e.* assay buffers without either a test sample or reference compound which corresponded to the MIN signal response (0% activity). MAX and MIN controls were used for data analysis (RZ' calculation) and normalization of compound activity.

For data analysis the response value was calculated as the maximum signal of the kinetic trace after the injection minus the median of the points before injection and multiplied by the normalization factor (NF). The normalisation factor was the baseline mean of all wells in the plate divided by the baseline of a specific well.

The agonistic effect was expressed as a percentage of activity, with 100% activity being a result in which the response value of the test sample wells reached a level identical to the one of the MAX controls (Max Signal wells corresponded to reference agonist $EC_{100}$) and 0% activity being a result in which the response value of the test wells reached a level identical to the one of the MIN controls (Min Signal wells corresponding to assay buffer).

To calculate the % of activity of the sample the response value was normalized versus MAX signal controls and MIN Signal controls to obtain the activity % according to the following formula:

$$Activity\% = 100 * (\frac{x - <MIN>}{<MAX> - <MIN>})$$

The normalized data in percent activity were then used to perform curve fitting and $EC_{50}$ calculation corresponding to the concentration that gave 50% of activation of the receptor (within a curve fitting program within the data analysis software).

Results:

- Spilanthol: $EC_{50}$ = 3.55 X $10^{-5}$ M,
- Elemi oil: $EC_{50}$ = 3.28 X $10^{-5}$ M (assuming the molecular weight of elemi oil is the same as that of elemol: 222.4 g.$mol^{-1}$),
- Elemi resin: $EC_{50}$ = 6.67 X $10^{-5}$ M (assuming the molecular weight of elemi resin is the same as that of elemol: 222.4 g.$mol^{-1}$),
- Elemol (isolated from elemi oil, 80% pure): $EC_{50}$ = 1.56 X $10^{-5}$ M,
- Elemicin (isolated from elemi oil, 90% pure): $EC_{50}$ = 9.37 X $10^{-5}$ M,
- Lime oxide: $EC_{50}$ = 6.24 X $10^{-5}$ M (molecular weight: 290.5 g.$mol^{-1}$),
- Isopulegol (Coolact® P): $EC_{50}$ = 5.70 X $10^{-5}$ M,
- Methoxypropanediol (Coolact® 10): $EC_{50}$ = 2.00 X $10^{-4}$ M.

These results show that elemi oil, elemol, elemicin, lime oxide, isopulegol (Coolact® P) and menthoxypropanediol (Coolact® 10) are comparable agonists for TRPA1 to spilanthol (reference).

It can be seen that elemi oil, elemol, elemicin, lime oxide, isopulegol and menthoxypropanediol activate TRPA1. Compounds with comparable agonist activity on TRPA1 (isopulegol and menthoxypropanediol) nevertheless displayed significantly different perceived tingling sensation when smelt (see example 3).

*Method for Screening on Receptors TRPV1:*

For screening on TRPV1 CHO-K1 (Chinese Hamster Ovary) cells stably transfected with human TRPV1 were grown in a culture medium of: Dubelco's modification of Eagles Medium F-12 (1:1) mixture (Lonza Catalogue No BE04-687F/U1) 500 mL, 5 mL of 100 mM sodium pyruvate, 25 mL of 7.5% sodium bicarbonate, 6.5 mL of 1M hepes (4-(2-hydroxyethyl)-1-piperazineethanesufonic acid), 5 mL of 100X Penicillin/Streptomycin, 50 mL Fetal bovine serum, 0.25 mL of 10 mg/mL Puromycin, 0.5 mL of 100 mg/mL Zeocin.

The reference agonist for the TRPV1 assay was capsaicin.

Results:

- Capsaicin: $EC_{50}$ = 1.40 X $10^{-8}$ M,
- Vanillyl ethyl ether (Hotact® VEE): $EC_{50}$ = 2.15 X $10^{-4}$ M, and
- Vanillyl butyl ether (Hotact® VBE): $EC_{50}$ = 7.58 X $10^{-6}$ M.

These results show that vanillyl ethyl ether (Hotact® VEE) and vanillyl butyl ether (Hotact® VBE) are agonists for TRPV1, but weaker agonists for TRPV1 than capsaicin (reference).

Although the $EC_{50}$ for vanillyl ethyl ether and vanillyl butyl ether is less than that of capsaicin the first two nevertheless provided an improved warming perception when evaluated when smelt, i.e. without direct contact to the skin (see example 1).

**Claims**

1. A household product comprising a fragrance composition, in which the fragrance composition comprises from about 0.01 to less than 10% by weight of at least one chemaesthethic agent selected from the group consisting of: vanillyl ethyl ether (CAS 13184-86-6), vanillyl n-propyl ether (CAS 81995-38-2), vanillyl isopropyl ether (CAS 14193-29-4), vanillyl butyl ether (CAS 82654-98-6), elemol (CAS 8024-27-9), elemicin (CAS 487-11-6), lime oxide (CAS 73018-51-6), ocimene quintoxide (CAS 7416-35-5), 2-isopropenyl-5-methyl-5-vinyltetrahydrofuran (CAS 13679-86-2) and isopulegol (CAS 7786-67-6 and CAS 89-79-2), based on the total weight of the fragrance composition.

2. The household product of claim 1, in which the fragrance composition comprises from about 0.01 to about 5% by weight of at least one chemaesthethic agent selected from the group consisting of: vanillyl ethyl ether (CAS 13184-86-6), vanillyl n-propyl ether (CAS 81995-38-2), vanillyl isopropyl ether (CAS 14193-29-4), vanillyl butyl ether (CAS 82654-98-6), elemol (CAS 8024-27-9), elemicin (CAS 487-11-6), lime oxide (CAS 73018-51-6), ocimene quintoxide (CAS 7416-35-5), 2-isopropenyl-5-methyl-5-vinyltetrahydrofuran (CAS 13679-86-2) and isopulegol (CAS 7786-67-6 and CAS 89-79-2), based on the total weight of the fragrance composition.

3. The household product of claim 1 or claim 2, in which the chemaesthetic agent is a warming agent selected from the group consisting of vanillyl ethyl ether (CAS 13184-86-6) vanillyl n-propyl ether (CAS 81995-38-2), vanillyl isopropyl ether (CAS 14193-29-4), vanillyl butyl ether (CAS 82654-98-6), and mixtures thereof.

4. The household product of claim 3, in which the warming agent is combined with at least one complementary compound selected from the group consisting of: 2-ethoxy-4-(hydroxymethyl) phenol (CAS 4912-58-7), vanillin (CAS 121-33-5), ethyl vanillin (CAS 121-32-4), vanillyl isobutyrate (CAS 20665-85-4), veltol (CAS 118-71-8), veltol plus (CAS 4940-11-8), cinnamaldehyde (CAS 104-55-2), vertenex (CAS 32210-23-4), coumarin (CAS 91-64-5), orbitone® (CAS 55464-57-2, CAS 68155-66-8 and CAS 68155-67-9) and bacdanol (CAS 28219-61-6 and CAS 164203-46-7).

5. The household product of claim 1 or claim 2, in which the chemaesthetic agent is a tingling agent selected from the group consisting of elemol (CAS 8024-27-9), elemicin (CAS 487-11-6), lime oxide (CAS 73018-51-6), ocimene quintoxide (CAS 7416-35-5), 2-isopropenyl-5-methyl-5-vinyltetrahydrofuran (CAS 13679-86-2), isopulegol (CAS 7786-67-6 and CAS 89-79-2), and mixtures thereof.

6. The household product of claim 5, in which the tingling agent is combined with at least one complementary compound selected from the group consisting of: citral (CAS 5392-40-5), 1,4-cineole (CAS 470-67-7), gamma-terpinolene (CAS 69073-38-7) and (+)-nootkatone (CAS 4674-50-4).

7. The household product of any one of claims 1 to 6, which is an air freshener dispenser device.

8. The air freshener dispenser device of claim 7, which is a passive evaporative device selected from sprayers, diffusers, gels, candles, waxes, wax-melts, pot-pourri, impregnated papers and laminated cardboard.

9. The air freshener dispenser device of claim 8, which is an electrically powered air freshener dispenser device, and preferably a plug or a piezo-electric device.

10. The electrically powered air freshener dispenser device of claim 9, which comprises a heating element.

11. The household product of any one of claims 1 to 7, which is a floor cleaner.

12. The household product of any one of claims 1 to 7, which is a kitchen or bathroom surface cleaner.

13. The household product of any one of claims 1 to 7, which is a toilet rim block or a toilet cistern block.

14. Use of at least one of vanillyl ethyl ether (CAS 13184-86-6), vanillyl n-propyl ether (CAS 81995-38-2), vanillyl isopropyl ether (CAS 14193-29-4), vanillyl butyl ether (CAS 82654-98-6), or of a household product as defined in any one of claims 3, 4 and 7 to 13, as an agent for delivering a perceived warming sensation through the air.

15. Use of at least one of elemol (CAS 8024-27-9), elemicin (CAS 487-11-6), lime oxide (CAS 73018-51-6), ocimene quintoxide (CAS 7416-35-5), 2-isopropenyl-5-methyl-5-vinyltetrahydrofuran (CAS 13679-86-2), isopulegol (CAS 7786-67-6 and CAS 89-79-2), or of a household product as defined in any one of claims 5 to 13, as an agent for delivering a perceived tingling sensation through the air.

16. A method for delivering a perceived warming and tingling sensation through the air which comprises dispersing:

- at least one warming agent selected from the group consisting of: vanillyl ethyl ether (CAS 13184-86-6), vanillyl n-propyl ether (CAS 81995-38-2), vanillyl isopropyl ether (CAS 14193-29-4), vanillyl butyl ether (CAS 82654-98-6), simultaneously with

- at least one tingling agent selected from the group consisting of: elemol (CAS 8024-27-9), elemicin (CAS 487-11-6), lime oxide (CAS 73018-51-6), ocimene quintoxide (CAS 7416-35-5), 2-isopropenyl-5-methyl-5-vinyltetrahydrofuran (CAS 13679-86-2), isopulegol (CAS 7786-67-6 and CAS 89-79-2),

or a household product as defined in any one of claims 1 to 13 comprising both a warming and a tingling agents, into the air.

17. A method for delivering a perceived warming sensation through the air which comprises dispersing at least one of vanillyl ethyl ether (CAS 13184-86-6), vanillyl n-propyl ether (CAS 81995-38-2), vanillyl isopropyl ether (CAS 14193-29-4), vanillyl butyl ether (CAS 82654-98-6), or a household product as defined in any one of claims 3, 4 and 7 to 13, into the air.

18. A method for delivering a perceived tingling sensation through the air which comprises dispersing at least one of elemol (CAS 8024-27-9), elemicin (CAS 487-11-6), lime oxide (CAS 73018-51-6), ocimene quintoxide (CAS 7416-35-5), 2-isopropenyl-5-methyl-5-vinyltetrahydrofuran (CAS 13679-86-2), isopulegol (CAS 7786-67-6 and CAS 89-79-2), or a household product as defined in any one of claims 5 to 13, into the air.

**Warming agents**

**FIG.1**

**Tingling agents**

**FIG.2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1315626 A
- US 6267297 B
- US 4814212 A
- US 6036925 A
- US 20070122373 A
- US 9890567 B
- US 5773410 A
- JP 54067040 A
- JP 61009293 A

- US 6123935 A
- EP 0462643 A
- GB 2178442 A
- US 8658588 B
- US 4874536 A
- WO 0240792 A
- EP 0775741 A
- WO 0194520 A

### Non-patent literature cited in the description

- *CHEMICAL ABSTRACTS,* 81995-38-2
- *CHEMICAL ABSTRACTS,* 14193-29-4
- *CHEMICAL ABSTRACTS,* 82654-98-6
- *CHEMICAL ABSTRACTS,* 8024-27-9
- *CHEMICAL ABSTRACTS,* 487-11-6
- *CHEMICAL ABSTRACTS,* 73018-51-6
- *CHEMICAL ABSTRACTS,* 7416-35-5
- *CHEMICAL ABSTRACTS,* 13679-86-2
- *CHEMICAL ABSTRACTS,* 7786-67-6
- *CHEMICAL ABSTRACTS,* 89-79-2
- **T. MC DONALD et al.** Chemesthesis: Chemical Touch in Food and Eating. Wiley-Blackwell, March 2016
- *CHEMICAL ABSTRACTS,* 4912-58-7
- *CHEMICAL ABSTRACTS,* 121-33-5
- *CHEMICAL ABSTRACTS,* 121-32-4
- *CHEMICAL ABSTRACTS,* 20665-85-4
- *CHEMICAL ABSTRACTS,* 118-71-8

- *CHEMICAL ABSTRACTS,* 4940-11-8
- *CHEMICAL ABSTRACTS,* 104-55-2
- *CHEMICAL ABSTRACTS,* 32210-23-4
- *CHEMICAL ABSTRACTS,* 91-64-5
- *CHEMICAL ABSTRACTS,* 55464-57-2
- *CHEMICAL ABSTRACTS,* 68155-66-8
- *CHEMICAL ABSTRACTS,* 68155-67-9
- *CHEMICAL ABSTRACTS,* 28219-61-6
- *CHEMICAL ABSTRACTS,* 164203-46-7
- *CHEMICAL ABSTRACTS,* 5392-40-5
- *CHEMICAL ABSTRACTS,* 470-67-7
- *CHEMICAL ABSTRACTS,* 69073-38-7
- *CHEMICAL ABSTRACTS,* 4674-50-4
- **S. ARCTANDER.** Perfume Flavors and Chemicals. 1969, vol. I and II
- The Merck Index. Merck & Co., Inc,
- **E. GUENTHER.** The Essential Oils. 1949
- Surfactant Science Series. *Liquid Detergents chapter on Speciality Liquid Household Surface Cleaners,* vol. 67, 479
- *CHEMICAL ABSTRACTS,* 13184-86-6
- **S. KOSUGE et al.** *J. Agric. Chem. Soc.,* 1962, vol. 36, 51
- **J. PAULRAJ et al.** *Advances in Pharmacological Sciences,* vol. 2013, 22